# EUROPEAN PATENT SPECIFICATION

(11) **EP 2 350 894 B1**
(45) Date of publication and mention of the grant of the patent: **03.07.2019**
(21) Application number: 09741000.5
(22) Date of filing: 10.09.2009
(51) Int. Cl.: G16H 50/70

(54) **METHODS AND DEVICES FOR TAILORING A BOLUS DELIVERY PATTERN**
VERFAHREN UND EINRICHTUNGEN ZUM ZURECHTSCHNEIDEN EINES BOLUS-VERABREICHUNGSMUSTERS
PROCÉDÉS ET DISPOSITIFS POUR ADAPTER UN MODÈLE D'ADMINISTRATION À INJECTION RAPIDE

(30) Priority: 11.09.2008 US 96087 P
(43) Date of publication of application: 03.08.2011
(73) Proprietor: Roche Diabetes Care GmbH, 68305 Mannheim (DE); F. Hoffmann-La Roche AG, 4070 Basel (CH)
(72) Inventor: YODFAT, Ofer, Modi'in 71725 (IL); SHAPIRA, Gali, 34558 Haifa (IL); LEVY, Aharon, 76868 Moshav Beit Hanan (IL)
(74) Representative: Evens, Paul Jonathan
(86) International application number: PCT/IL2009/000888
(87) International publication number: WO 2010/029551

(56) References cited:
- US-A1- 2007 179 434
- US-A1- 2008 183 060
- T. R. Nansel ET AL: "Effect of Varying Glycemic Index Meals on Blood Glucose Control Assessed With Continuous Glucose Monitoring in Youth With Type 1 Diabetes on Basal-Bolus Insulin Regimens", DIABETES CARE, vol. 31, no. 4, 1 April 2008 (2008-04-01), pages 695-697, XP055391916, US ISSN: 0149-5992, DOI: 10.2337/dc07-1879
- FOSTER-POWELL K ET AL: "International table of glycemic index and glycemic load values: 2002", THE AMERICAN JOURNAL OF CLINICAL NUTRITION, AMERICAN SOCIETY FOR NUTRITION, US, vol. 76, no. 1, 1 July 2002 (2002-07-01), pages 5-56, XP002369785, ISSN: 0002-9165

## Description

### CROSS-REFERENCE TO RELATED APPLICATIONS

The present disclosure claims priority to U.S. Provisional Patent Application No. 61/096,087, entitled "Method and a Device for Tailoring a Bolus Delivery Pattern in a Drug Delivery Device," filed on September 11, 2008.

### FIELD

Methods, devices and systems for sustained delivery of therapeutic fluids (e.g., insulin) to a user/patient are described. In particular, the present disclosure includes embodiments directed toward portable devices and methods for implementing these devices for tailoring a bolus delivery pattern of therapeutic fluid. More particularly, skin securable insulin dispensing devices and methods for implementing these devices for tailoring a bolus delivery pattern according to the nutritional characteristics (e.g., amount of carbohydrates, glycemic index or fat content) of an individuals' dietary intake are provided.

### BACKGROUND

Diabetes mellitus is a disease of major global importance, increasing in frequency at almost epidemic rates, with 170 million known cases worldwide in 2006. This number is expected to at least double over the next 10-15 years. Diabetes is characterized by a chronically raised blood glucose concentration (known as hyperglycemia) due to a relative or absolute lack of the pancreatic hormone, insulin. In a healthy (i.e., non-diabetic) pancreas, beta cells located in the islets of Langerhans continuously produce and secrete insulin according to blood glucose levels and maintain near constant glucose levels in the body.

Much of the burden of the disease to the patient and health care resources is due to the long-term tissue complications that affect both small blood vessels (i.e., microangiopathy causing eye, kidney and nerve damage) and large blood vessels (causing accelerated atherosclerosis with increased rates of coronary heart disease, peripheral vascular disease and stroke). The Diabetes Control and Complications Trial ("DCCT") has demonstrated that development and progression of the chronic complications of diabetes are greatly related to the degree of altered glycemia as quantified by determinations of glycohemoglobin (HbA1c). See, e.g., DCCT Trial, New Eng. J. Med. 1993, 329: 977-986; UKPDS Trial, Lancet 1998, 352: 837- 853; BMJ 1998, 317, (7160): 703-13; EDIC Trial, New Eng. J. Med. 2005, 353, (25): 2643-53. Thus, maintaining euglycemia by frequent glucose measurements and making corresponding adjustments of insulin delivery is of utmost importance.

Insulin pumps can deliver rapid acting insulin 24 hours a day through a catheter placed under the skin. The total daily insulin dose includes basal and bolus doses. Insulin basal doses may be delivered continuously (or periodically) over a 24-hour period and facilitate keeping the blood glucose levels in an acceptable insulin range between meals and overnight. Diurnal basal rates can be pre-programmed or manually changed according to various daily activities.

Insulin bolus doses may be delivered before or after meals to counteract carbohydrate loads and/or during episodes of high blood sugar levels. The amount of insulin in a bolus dose may depend on several parameters:
- the amount of carbohydrates in the meal to be consumed;
- the carbohydrate-to-insulin ratio ("CIR"), i.e. the amount of carbohydrates balanced by one unit of insulin;
- insulin sensitivity ("IS"), i.e. the blood glucose value lowered by one unit of insulin;
- the current blood glucose level ("CBG");
- the target blood glucose level ("TBG"), i.e. the desired blood glucose level. TBG for most people suffering from diabetes is in the range of 80-130 mg/dL; and
- the remaining insulin ("RI", also referred-to as "residual insulin"), i.e. the amount of stored insulin remaining active in the body after earlier bolus doses have been delivered. This parameter is relevant when there is a short time interval between delivering consecutive bolus doses {e.g., intervals of less than 5 hours).

The insulin amount in a delivered bolus dose can be determined using equations that incorporate the above parameters, as described, for example, in U.S. Patent No. 6,936,029 to Mann et al. The insulin amount may also be determined using a method as described in co-owned, co-pending U.S. publication no. 2008/0234663. It should be noted that the above methods for determining the bolus dose to be delivered generally involve assessing a bolus dose but do not address the manner and/or pattern for delivering the dose.

A bolus dose delivery pattern may refer to a rate or rates at which a bolus dose is delivered over time. For example, a meal containing purely carbohydrates may require very fast delivery at one constant delivery rate. A meal containing carbohydrates and fat (e.g., a pizza meal) may require a fast delivery rate for a short time followed by a slower delivery rate for an extended time period (e.g., two delivery rates).

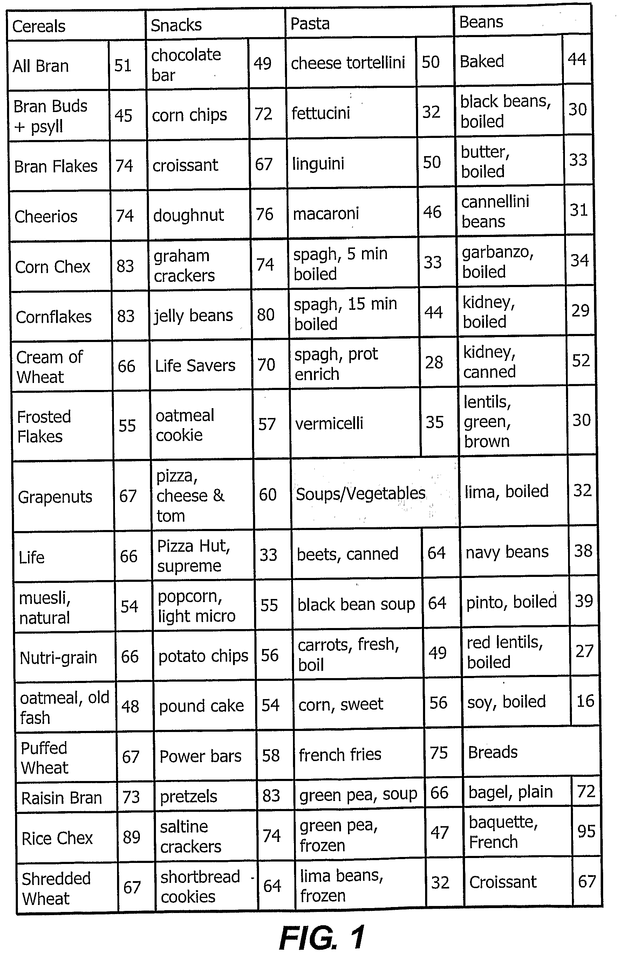

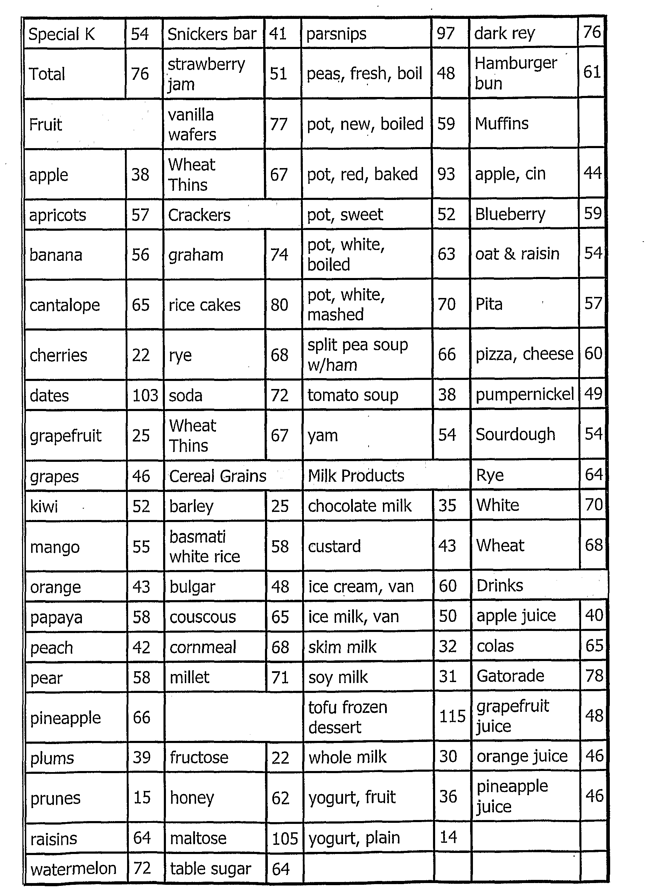

Certain parameters, such as the glycemic index ("GI") or fat content of the consumed food, can influence carbohydrate absorption and consequently bolus delivery duration and pattern. The GI can be described in terms of a ranking system based on the type of carbohydrates contained in food and their effect on blood glucose levels. According to the GI, glucose (the fastest-acting carbohydrate) is given a value of 100, and the other carbohydrates are ranked relative to that value. Ripeness, cooking time, fiber, and fat content in the food can all impact the GI of certain foods. As shown in FIG. 1, different foods have different GI's.

A low GI food will release glucose more slowly and steadily. A high GI food will release glucose more rapidly. A meal containing a carbohydrate load having high GI will thus require immediate insulin delivery to counteract the rapidly absorbed carbohydrates. Conversely, a meal containing a carbohydrate load having a low GI will require insulin delivery over an extended time period to counteract the slowly absorbed carbohydrates. In addition, changes in blood glucose levels following food consumption and/or delivery of an insulin dose may vary from one diabetic patient to another.

Currently, most insulin pumps enable the user to program a bolus dose delivery pattern before delivery. The most common programmable bolus dose delivery patterns include:
- immediate ("normal") - the entire bolus dose is delivered at the fastest pump delivery rate;
- extended - the entire bolus dose is delivered over a period of time (e.g., 30 min, to 8 hours) at a constant rate; or
- combined ("dual wave") - some of the bolus dose is delivered immediately and the rest of the dose, is delivered as an extended bolus dose. The ratio between the immediate portion and the extended portion may be selected by the patient as described in U.S. Patent No. 6,852,104 to Blomquist.

In conventional systems, a user may program a bolus dose delivery pattern by inputting data, such as the duration of the bolus dose delivery (for an extended bolus or extended bolus portion of a combined bolus) and/or the ratio between the immediate bolus portion and extended bolus portion (for a combined bolus).

A drawback associated with users preprogramming bolus dose delivery information is the user's inability to estimate the appropriate bolus delivery duration and pattern (e.g., immediate, extended or combined). Often, a user's decision about the bolus dose delivery pattern can be arbitrary and based merely on intuition. In addition, mechanical capabilities of the pump, which can enable minute-by-minute variability in delivery rate, is not fully being exploited, as the most "complex" bolus dose delivery pattern currently offered to users is composed of merely two phases in the combined or "dual wave" bolus dose.

US 2008/183060A relates to a system and method for controlling and monitoring a diabetes-management system through the use of a model that predicts or estimates future dynamic states of glucose and insulin from variables such as insulin delivery. US 2007/179434A relates to a system for determining drug administration information and discloses a device according to the pre-amble of appended independent claim 1.

### SUMMARY

In accordance with a first aspect of the present invention, there is provided a therapeutic fluid dispensing device as defined in appended independent claim 1. Embodiments of the present invention are defined in appended claims which depend on independent claim 1. In accordance with a second aspect of the present invention, there is provided a method of tailoring a bolus delivery pattern for a user as defined in appended independent claim 9. Devices and methods for providing a user with a bolus delivery pattern (hereinafter "bolus delivery pattern") tailored to the user's physiology are described. In some embodiments, the physiological parameters may correspond to at least one of a heart rate, a ventilation rate, a body temperature, insulin absorption characteristics (e.g., rate), physical activity (e.g., exercise of the user per period of time), cannula insertion site or fat tissue characteristics of the patient. In some embodiments, the devices and methods may provide a diabetes patient with better treatment and care by tailoring bolus delivery patterns to emulate a non-diabetic person's average blood glucose ("BG") response to food consumption. Some methods and devices may provide for tailoring bolus delivery patterns of a drug. Method aspects include tailoring a bolus delivery pattern of a drug by comparing a non-diabetic person's average BG response over time (hereinafter "healthy response") to that of the user after consuming a similar meal and correcting the user's response to match the healthy response. In method embodiments, the bolus delivery pattern may be tailored for a user by analyzing the physiological post-prandial increase in BG observed in one or more non-diabetic individuals. Such methods contrast with the objectives and functionality of pumps currently available to users, which bring users to fasting target blood glucose levels without considering any physiological post-prandial increases in BG observed in a non-diabetic individual.

Method embodiments for providing a user with tailored bolus delivery patterns may include using graphs representing the healthy response to different meals (e.g., 4-5 meal types of different GPs). The "y" axis of the graph may represent the measured BG (mg/dL) and the "x" axis may represent the time that has elapsed since the meal (e.g., in minutes). Subsequently, the area-under-the-curve (hereinafter "AUC") of the average healthy response ("AUCₕ") may be calculated. The AUC may be presented in units of [min^{∗}mg/dL]. BG measurements may be performed at constant time intervals (e.g., every 20 min.) to determine BG versus time curves. When the user subsequently consumes similar meals (e.g., 4-5 meal types), an adequate bolus dose (e.g., based on adequate carbohydrate load assessment and the user's CIR), preferably an extended bolus (e.g., 60 min.) in some embodiments, can be administered to counteract the content of meals, starting at a predefined period before the meal (e.g., 30 min.). Mathematical relationships (which may be represented as graphs) representing the user's response to the different meals, after the bolus delivery may be provided. The BG may be measured periodically (e.g., every 20 min.) to obtain the mathematical relationship (e.g., a graph). The AUC of the user's response ("AUCᵤ") can then also be computed. In some embodiments, the optimal, or near optimal, dose and dose distribution rate over a particular period of time (i.e., the delivery rate) can be determined from the difference between the AUC of the user and the AUC of a healthy response, i.e., AUCᵤ-AUCₕ.

In some embodiments, a drug delivery device may be implemented to perform a bolus delivery pattern tailoring operation to adjust a bolus delivery rate to a predefined meal or meal type. Some embodiments of the device may include a processor configured to control the bolus dose delivery pattern tailoring operation. In some embodiments, the device may also include a memory configured to store one or more bolus delivery patterns according to one or more meals or meal types. A display for providing graphical representations of the tailored bolus delivery pattern may be included on the device according to some embodiments. Some device embodiments may also have a user interface to receive a meal or meal type input from the user, where the meal or meal type input corresponding to a tailored graphical bolus delivery pattern. In some embodiments, the drug may be insulin and the drug delivery device may be an insulin pump.

In some embodiments, a device that can monitor BG levels and dispense bolus doses according to a tailored bolus delivery pattern is provided. In some embodiments, a device that continuously (or periodically) monitors BG levels and concomitantly delivers bolus doses according to a tailored bolus delivery pattern is provided.

In some arrangements, a device that includes a dispensing patch unit composed of two parts - a reusable part and disposable part - may be provided. The reusable part may contain relatively expensive components and the disposable part may contain relatively inexpensive components, thus providing a low cost product for the user and an economically viable product for the manufacturer and/or payer (e.g., healthcare insurance providers). In some arrangements, the unit may be skin securable. The device may also infuse insulin. The device may further be implemented to perform a procedure for tailoring an insulin bolus delivery pattern for a user. The dispensing patch unit may be attached to the skin directly or via a cradle unit. In some arrangements, the device may be remotely controlled. Some arrangements provide a device with a dispensing patch unit that can be disconnected and/or reconnected to a user. The device may implement a procedure for tailoring a bolus delivery pattern, according to some arrangements. Some arrangements disclosed herein may also provide a device having a miniature skin securable dispensing patch unit that can continuously dispense insulin and monitor BG levels. This device may also implement a procedure for tailoring a bolus delivery pattern.

Some arrangements disclosed herein may provide a semi-closed loop system that can monitor BG levels and dispense bolus doses according to sensed glucose levels and a tailored bolus delivery pattern. The system may be implemented in a single miniature device that may be discreet and cost-effective for users and/or payers (e.g., health insurance companies).

In some arrangements, the method(s) for providing a suitable bolus delivery pattern can be based on re-establishing tailored bolus profiles, for example, by comparing a user's BG responses to certain meals to a profiled healthy response to the same meals and providing an appropriate pre-established tailored bolus delivery pattern in subsequent meals of the same type. The data that determines the meal type may include at least the GI of the food to be consumed. In some arrangements, the tailored bolus delivery pattern can be selected from a plurality of pre-established bolus delivery patterns, with each pattern allocated to a different GI range (e.g., GK55, 56<GI<69, GI>70). The GI of the food may be designated as a qualitative, descriptive parameter ("QDP") (e.g., high GI, intermediate GI, low GI or combined GI). Presenting the GI of the food as a qualitative parameter instead of a number may be particularly convenient for young children.

In some arrangements, the user can accept a recommended bolus delivery pattern and have a bolus dose delivered accordingly. In some arrangements, the selected bolus delivery pattern may be delivered without acceptance by the user. For example, the user can be notified prior to bolus dose delivery and can suspend the delivery or select an alternative bolus delivery pattern.

The method arrangements of providing a tailored bolus delivery pattern can be implemented in an insulin infusion device having a dispensing patch unit and a remote control unit. In some arrangements, the remote control unit may have a glucose sensing apparatus (e.g., a glucometer). The method arrangements of providing a tailored bolus delivery pattern can also be implemented in the remote control unit of the insulin infusion device, a reusable part of the dispensing patch unit of the device or both. In some arrangements, the dispensing patch unit of these method arrangements may continuously or periodically monitor BG levels and can concomitantly deliver insulin into the body. The dispensing patch unit may also include a reusable part and a disposable part. In some arrangements, the insulin dispensing and glucose sensing capabilities can be combined into a semi-closed loop system and a processor-controller apparatus may regulate the delivery of insulin bolus doses according to the sensed glucose concentration. In preferred arrangements, the bolus doses may be delivered in accordance with a tailored bolus delivery pattern.

In some arrangements, the recommended tailored bolus delivery pattern can be chosen from a plurality of pre-established tailored bolus delivery patterns based on the nutritional composition (e.g., the percentage of fat, protein and/or carbohydrates) of the food consumed (or to be consumed) by the user. In some arrangements, the dispensing patch unit may deliver a bolus dose in accordance with a tailored bolus delivery pattern selected from a plurality of pre-established bolus delivery patterns and also receive glucose levels readings. In some arrangements, the dispensing patch unit may continuously and/or periodically monitor glucose levels and deliver a bolus dose in accordance with a delivery pattern selected from a plurality of pre-established bolus delivery patterns.

Some method arrangements may provide a method for therapeutic fluid delivery. In some arrangements, the method may include determining a bolus delivery pattern based on a specified type of dietary intake to be consumed by a user (which may also be referred to as "the patient") and delivering a therapeutic fluid into the user's body based, at least in part, on the determined bolus delivery pattern. arrangements of the method may include determining the bolus delivery pattern by selecting the bolus delivery pattern from one or more bolus delivery patterns based on the specified type of dietary intake, where at least some of the one or more bolus delivery patterns may be determined based, at least in part, on dietary intake similar to the specific dietary intake of the user and corresponding blood glucose responses of one or more non-diabetic individuals that have consumed dietary intake similar to the specific dietary intakes of the user. The method may further include determining bolus delivery patterns based, at least in part, on users' BG responses after consuming dietary intake similar to the specific dietary intake of the user.

Determining a bolus delivery pattern includes computing a difference between the AUC of a graphical representation of a user's response to a dietary intake and the AUC of a graphical representation of the healthy response for the same intake.

The one or more bolus delivery patterns may include multiple bolus delivery patterns that each correspond to an associated meal type. The multiple bolus delivery patterns may include, for example, 2-10 patterns that each correspond to a different meal type. In some embodiments disclosed herein, the plurality of bolus delivery patterns may be stored in an electronic storage device.

In some arrangements, the methods disclosed herein may further include adjusting the determined bolus delivery pattern based on pharmacodynamics characteristics of the therapeutic fluid. Adjusting the determined bolus delivery pattern based on the pharmacodynamics characteristics of the therapeutic fluid may include time-shifting the determined bolus delivery pattern. In some arrangements, the method may include receiving information from the patient regarding the type of dietary intake. Some method arrangements may also include pumping insulin stored in a reservoir in a insulin infusion device into the user, graphically representing the bolus delivery pattern and/or measuring BG level of the patient.

In some arrangements, delivering the therapeutic fluid into a user based, at least in part, on the determined bolus delivery pattern may further include delivering the therapeutic fluid into a user based, at least in part, on the determined bolus delivery pattern and on the measured blood glucose level. Some methods may include measuring the blood glucose level using a glucometer or continuous glucose monitor.

Some device arrangements provide a therapeutic fluid dispensing device that delivers therapeutic fluid into a body of a user is disclosed. The device may include a controller that determines a bolus dose delivery pattern based on a specified type of dietary intake to be consumed by the user and an infusion pump to deliver the therapeutic fluid into a body of the user based, at least in part, on the determined bolus delivery pattern. Furthermore, arrangements of the device may include any of the features described in relation to the disclosed methods, as well as a user-input interface to receive at least information regarding the type of dietary intake, a reservoir in fluid communication with the infusion pump and configured to contain the therapeutic fluid, a display to represent graphically the determined bolus delivery pattern and a blood glucose sensor to measure the BG level of the patient. In some arrangements, the infusion pump may be configured to deliver therapeutic fluid into a user based, at least in part, on a determined bolus delivery pattern and a measured BG level. In some arrangements, the blood glucose sensor may include a glucometer that may be disposed in a remote control unit and further include the remote control unit. In some arrangements, the blood glucose sensor may include a continuous glucose monitor that may be disposed in a dispensing patch unit that may further include an infusion pump.

Some arrangements may include a method for tailoring a bolus delivery pattern to a patient that includes retrieving data related to a healthy response. The data may comprise one or more records, wherein each record includes a meal type and a corresponding first set of analyte concentration levels for a first period of time occurring after a meal has been consumed by a healthy individual. Such method arrangements may also include (i) initiating a bolus dose administration to the body of the patient, (ii) receiving a second set of analyte concentration levels corresponding to a second period of time, the second period of time occurring after the meal has been consumed by the patient, (iii) correlating the first set of analyte concentration levels and second set of analyte concentration levels and/or (iv) determining a bolus delivery pattern associated with the meal consumed by the patient, based on the correlation between the first set of analyte concentration levels and the second set of analyte concentration levels. Some arrangements may further comprise storing the determined bolus pattern in a memory. In some arrangements, at least one of the first set of analyte concentration levels or the second set of analyte concentration levels may be received from a continuous glucose monitor (CGM). Moreover, such method aspects may comprise visually displaying at least one of the first set of analyte concentration levels or the second set of analyte concentration levels.

In some methods arrangements, visually displaying at least one of the first set of analyte concentration levels or the second set of analyte concentration levels may comprise displaying a background image corresponding to the first set of analyte concentration levels and displaying a primary image corresponding to the second set of analyte concentration levels, wherein the primary image may be superimposed on the background image. In other arrangements, methods aspects may include visually displaying the determined bolus delivery pattern and/or administering therapeutic fluid to the body of the patient according to the determined bolus delivery pattern. Some arrangements may further comprise repeating the initiating, receiving, correlating and determining for different meal types. Some method aspects may incorporate a therapeutic fluid delivery device having (i) a reservoir retaining the therapeutic fluid, (ii) a driving mechanism dispensing the therapeutic fluid from the reservoir to the body of the patient, (iii) a controller controlling the dispensing of the therapeutic fluid, the controller being capable of correlating the first set of analyte concentration levels and the second set of analyte concentration levels, (iv) a memory storing at least one of the data related to a healthy response, the first set of analyte concentration levels, the second set of analyte concentration levels or the determined bolus delivery pattern and/or (v) a screen displaying at least one of the first set of analyte concentration levels, the second set of analyte concentration levels or the determined bolus delivery pattern. Some methods may include receiving a meal type.

In some arrangements, the disclosed methods may include dispensing a therapeutic fluid to the body of a patient according to a meal type. Such arrangements may include receiving a meal type and a content of a dietary intake to be consumed by the patient. In some arrangements, the methods may also include retrieving from a memory a pre-determined bolus pattern delivery corresponding to the meal type, determining a bolus amount of the therapeutic fluid corresponding to the content of the intake and/or dispensing the bolus amount of the therapeutic fluid to a body of a patient according to the retrieved bolus delivery pattern. Some method arrangements may include visually presenting the retrieved bolus delivery pattern. Furthermore, the meal type may be associated with a food database. In some arrangements, the content of the dietary intake may be one or more of a carbohydrate load, a GI, a fat content or a fiber content. In some arrangements, the bolus delivery pattern may correspond to one or more parameters selected from a group consisting of a CIR of the patient, an IS of the patient, a TBG of the patient, an RI of a patient and a physiological parameter of the patient.

Some device arrangements may include a graphic user interface of a drug delivery device for selecting a bolus delivery pattern. Some arrangements may include a first input element enabling the selection of a bolus amount to be delivered to a body of a patient, a second input element enabling the selection of a meal type, the meal type corresponding to a bolus delivery pattern stored in a memory of the drug delivery device and a third input element enabling initiation of drug delivery to the body of the patient corresponding to the bolus amount and bolus delivery pattern. In some arrangements, at least one of the first input element and second input element of the graphic user interface may have a scrolling functionality. The graphic user interface may further comprise a window associated with a food database that enables the selection of the meal type, according to some arrangements. The graphic user interface may also, in some arrangements, include at least one input element that visually presents at least one of a plurality of meal types, the selected meal type, the selected bolus amount, the selected bolus pattern and/or an analyte concentration level.

Arrangements of the graphic user interface may further include at least one output element visually representing a first set of analyte concentration levels, wherein the first set may correspond to the blood glucose response of a non-diabetic individual. Also, arrangements of the graphic user interface may further include at least one output element visually representing a second set of analyte concentration levels, wherein the second set of analyte concentration levels may correspond to the blood glucose response of the patient. In some arrangements, the graphic user interface may be configured to include at least one output element visually representing a background image corresponding to the first set of analyte concentration levels and a primary image corresponding to the second set of analyte concentration levels.

### BRIEF DESCRIPTION OF THE DRAWINGS

FIG. 1 is a table listing different types of foods and their respective GI.
FIG. 2 is a schematic diagram of an insulin infusion device comprising an insulin dispensing unit and a remote control unit that can be provided with a bolus delivery pattern tailoring functionality.
FIGS. 3a-c are graphs depicting exemplary bolus delivery patterns offered to users of currently available pumps.
FIG. 4 is a flow diagram of a procedure for providing a user with tailored bolus delivery patterns according to some embodiments described herein.
FIG. 5 shows graphs representing the healthy response and a particular user's response to a certain meal according to some embodiments described herein.
FIGS. 6a-d are diagrams of a user interface for selecting a bolus dose and tailored bolus delivery pattern.
FIGS. 7a-c are schematic diagrams of an insulin infusion device comprising an insulin dispensing unit, a remote control unit having a tailored bolus delivery pattern feature and a sensor.
FIGS. 8a-b are schematic diagrams of two configurations of an insulin infusion device comprising an insulin dispensing unit, a remote control unit having a tailored bolus delivery pattern feature and a continuous subcutaneous glucose monitor.

### DETAILED DESCRIPTION

Disclosed are methods and devices for providing tailored bolus delivery patterns of drugs (e.g., insulin). In some arrangements, a method for tailoring a bolus delivery pattern of a drug for a drug delivery device is described. This method may comprise pre-establishing meal- specific tailored bolus delivery patterns, storing the plurality of bolus delivery patterns in a memory, selecting an appropriate pre-established bolus delivery pattern in subsequent meals of the same type and adjusting a drug bolus delivery rate using the selected bolus dose delivery pattern. While the illustrative arrangements disclosed herein are described primarily in terms of determining glucose concentrations in the blood, the inventive methods and devices may be implemented to determine the concentration of other bodily analytes in other bodily tissues (e.g., ISF, or interstitial fluid) as well.

FIG. 1 is a table listing different types of foods and their respective GPs. A low GI food will release glucose more slowly and steadily. A high GI food generally causes a more rapid increase in BG levels. A meal containing a carbohydrate load having high GI would thus require insulin to be delivered immediately to counteract the rapidly absorbed carbohydrates. A meal containing a carbohydrate load having a low GI would require insulin to be delivered over a long period of time to counteract the slowly absorbed carbohydrates.

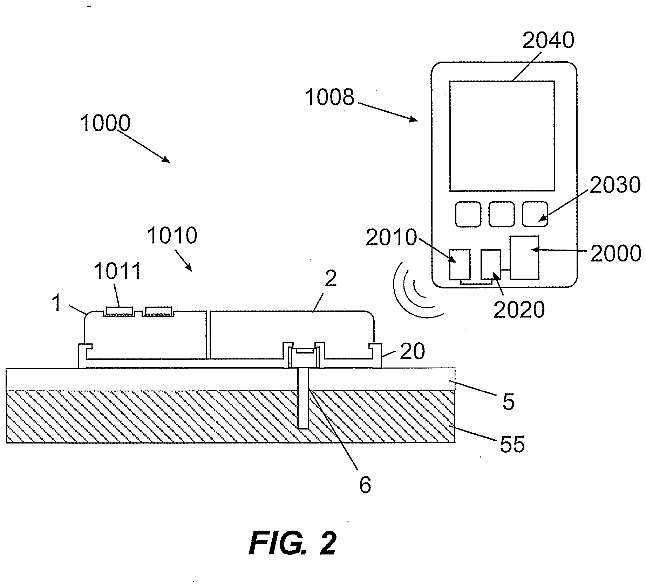

FIG. 2 shows a schematic diagram of an insulin infusion device 1000. The insulin infusion device 1000 may comprise a dispensing patch unit 1010. The unit 1010 may be secured to the user's skin 5 above the subcutaneous tissue 55. In some arrangements, a remote control unit 1008 may communicate with the dispensing patch unit 1010 to transmit (i) programming commands and/or instructions to and from the dispensing patch unit 1010, (ii) user input and output and/or (iii) acquired data.

In some arrangements, the dispensing patch unit 1010 may be detachable from the skin 5. The dispensing patch unit 1010 may also, according to some arrangements, receive a cannula 6 that penetrates the skin 5 to deliver therapeutic fluid (e.g., insulin) to the patient. The dispensing patch unit 1010 may be attached either directly to the skin 5 or to a cradle unit 20. In some arrangements, cradle unit 20 may be a low profile structure adherable to 'the skin 5 and enable connection and/or disconnection of the dispensing patch unit 1010 from the user. An exemplary arrangement of this type of an arrangement is described, for example, in co-owned, co-pending U.S publication no. 2008/0215035. Some arrangements of the dispensing patch unit 1010 may also contain inputs for controlling the unit 1010, including without limitation one or more buttons 1011, as shown in FIG. 2.

The dispensing patch unit 1010 may be contained in one or more housings. For example, in some arrangements, the dispensing patch unit 1010 may include a two-part housing having a reusable part 1 and a disposable part 2. This type of an arrangement is described in co- owned, co-pending U.S. Patent Application No. 11/397,115 and International Patent Application No. PCT/IL09/000388, published as WO 2009/125398.

In some arrangements, the remote control unit 1008 may be configured to provide a tailored bolus delivery pattern. For example, as shown in FIG. 2, the remote control unit 1008 may include a tailored bolus delivery pattern module 2000, a processor 2010, a memory 2020, an input interface 2030, a display 2040 and other indication devices (not shown), including without limitation audible and vibrational output devices. The input interface 2030 may, for example, facilitate operation of the tailored bolus delivery pattern module 2000 and/or programming of the dispensing patch unit 1010.

In some arrangements, providing a tailored bolus delivery pattern may allow for the selection and/or recommendation of a bolus delivery pattern from a plurality of pre-established tailored bolus delivery patterns stored and/or offered by a software-implemented application installed on a processor (not shown) included with the dispensing patch unit 1010. According to some arrangements, the tailored bolus delivery pattern module 2000 may be located in the reusable part 1 of the dispensing patch unit 1010.

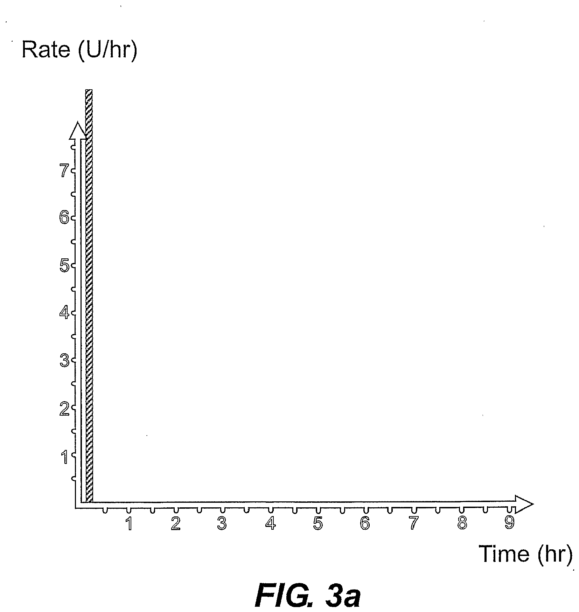

FIGS. 3a-c are graphical representations of exemplary bolus delivery patterns available for selection, recommendation and/or execution on conventional insulin pumps. The "y" axis represents the rate of bolus dose delivery {e.g., in Units/hours), and the "x" axis represents time {e.g., in hours). The area under the curve ("the AUC") is equal to the product of the rate and the time period and constitutes the bolus amount {e.g., in Units). The AUC is substantially the same in all the patterns depicted in FIGS 3a-c.

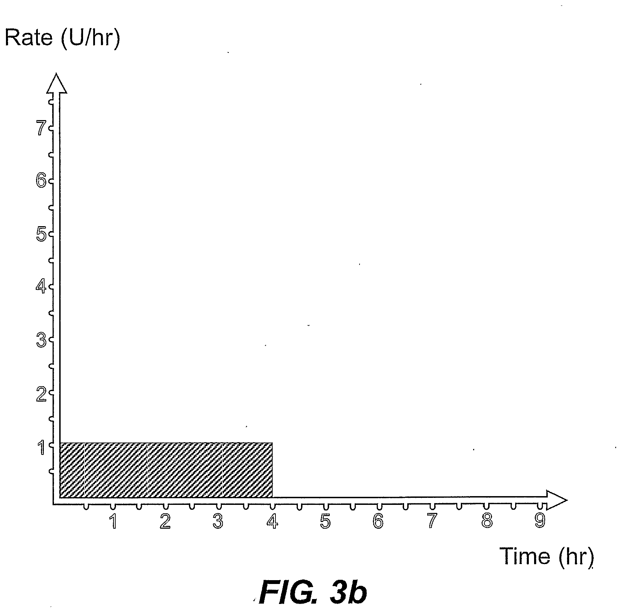

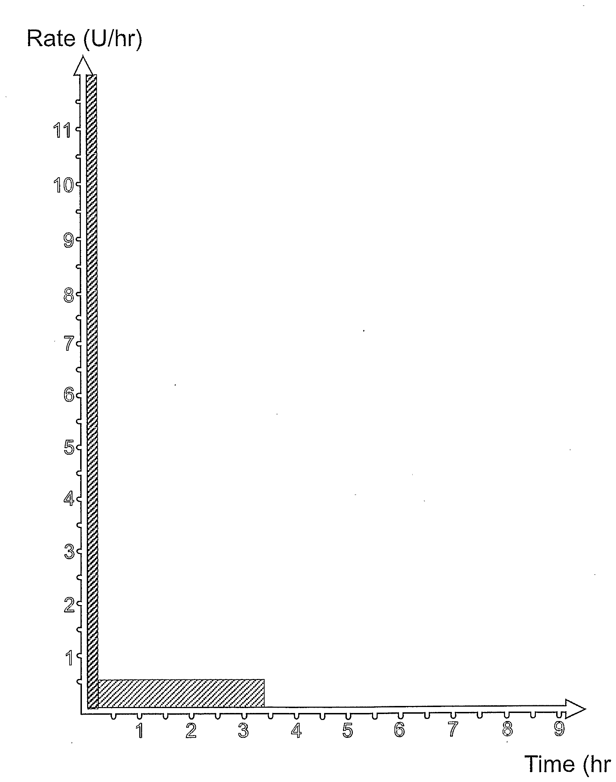

FIG. 3a depicts a bolus delivery pattern in which the entire bolus dose is delivered as rapidly as possible (i.e., immediate). FIG. 3b depicts a bolus delivery pattern in which the bolus dose is delivered evenly over a period of time (i.e., "extended"). In FIG. 3b, the bolus dose is delivered evenly over 4 hours. FIG. 3c depicts a bolus delivery pattern in which a portion of the bolus dose may be delivered immediately and the remaining dose delivered evenly over an extended period of time. This bolus delivery pattern may be called a combined or "dual wave" bolus dose. The user can generally set the duration of the extended portion of the bolus dose and its relative proportion. In the depicted pattern, and by way of example only, the immediate portion may comprise 60% of the total bolus dose, and the remaining 40% delivered over a period of time {e.g., 2-3 hours).

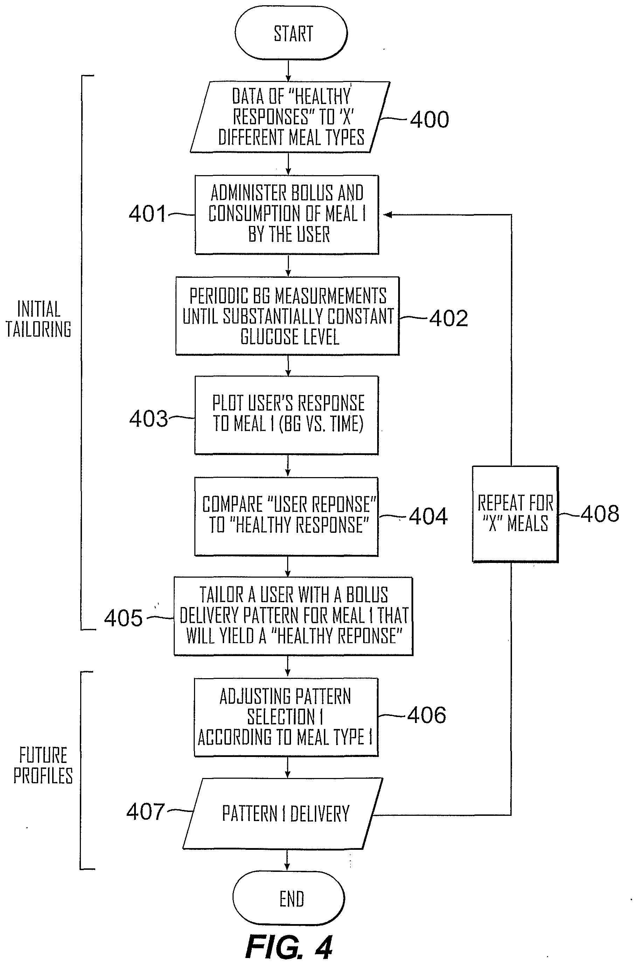

FIG. 4 shows a flow diagram of a procedure for providing tailored bolus delivery patterns according to some embodiments. Data of the healthy response to 'x' different meal types is provided at 400. In some embodiments, the notation 'x' may represent any number of different meal types {e.g., based on different GI's - high GI, medium GI, low GI and very low GI). A meal-specific tailoring procedure is performed at 401-405 according to some embodiments. For example, at 401 a user may consume a meal number 1 {e.g., one of the 'x' meals) and a bolus dose {e.g., a bolus dose delivered over a 60-minute time period) may be delivered to counteract the carbohydrate load of meal number 1. The bolus dose to be delivered may be computed using equations involving carbohydrate content and parameters including without limitation carbohydrate-to-insulin ratio ("CIR") and TBG, or target blood glucose, levels. The performance of such computations is described, for example, in U.S. Patent No. 6,936,029 to Mann *et al.* In some embodiments, the bolus dose level may be determined according to the selection procedure described, for example, in U.S. publication no. 2008/0234663.

Periodic (e.g., every 15 minutes) BG measurements may be performed at 402 (e.g., by a glucometer or continuous glucose monitoring ("CGM")) after consumption of the meal number 1 until a substantially constant BG level is reached (e.g., ±15mg/dL). In some embodiments, the user's response to the meal number 1 may be plotted at 403 as a BG versus time curve. The user's response to the meal number 1 may-be compared at 404 to the healthy response for a meal of the same type as the meal number 1. As will be described in greater details below, a bolus delivery pattern is then tailored at 405 to the user so that the user's response will substantially match, or nearly match, the healthy response.

In subsequent meals of the same type as meal number 1, the appropriate pre-established tailored bolus delivery pattern will be selected at 406, and delivered at 407. This procedure can be performed, as shown at 408, for any 'x' number of meals.

In some embodiments, the most suitable pre-established tailored bolus delivery pattern may be based on one or more stored tailored bolus delivery patterns based on a user's input of a meal. For example, a hamburger meal with French fries and soda would correspond to one of the stored tailored bolus delivery patterns. In some embodiments, the most suitable bolus delivery pattern may be recommended based on the user's input of meal type (e.g., a meal having a high, low or intermediate GI).

In some arrangements, the bolus dose may be delivered automatically according to a selected pre-established bolus delivery pattern corresponding to a specific meal. For example, when a user selects a meal from the meals list (e.g., a "pizza meal") the bolus dose may automatically be administered according to a corresponding tailored bolus delivery pattern. In some arrangements, the user can be recommended a particular bolus delivery pattern and may then accept or reject that recommendation. In some arrangements, the meal list can be presented alpha-numerically. The meal list may also be presented graphically on a display (e.g., display 2040 in FIG. 2) of the remote control unit 1008 or on a display (not shown) on the reusable part 1. According to some arrangements, the user may determine a bolus delivery pattern number (i.e., determine an 'x' number) tailored for selection of the bolus delivery pattern most appropriate for a specific meal.

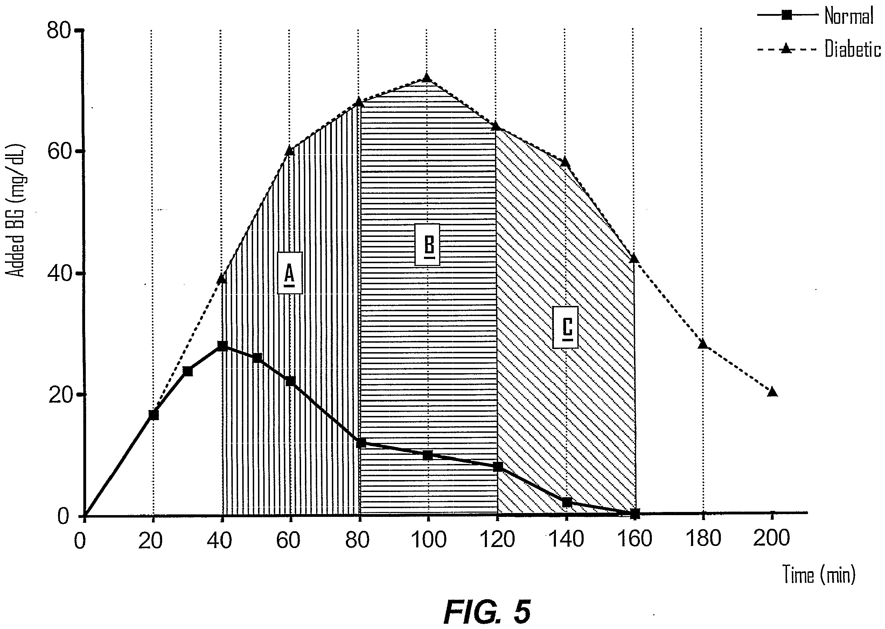

FIG. 5 shows graphs of a healthy response ("Normal" in FIG. 5) and the user's response ("Diabetic" in FIG. 5) to a certain meal type according to some embodiments. The "y" axis of the graph represents the added BG (mg/dL) (i.e., the glucose concentration added to the baseline fasting concentration due to meal consumption) and the "x" axis represents the time that has elapsed since the meal was consumed (e.g., in minutes).

In some embodiments, the procedure to tailor a bolus delivery pattern and/or provide the user with such a tailored bolus dose and pattern, based on a planned meal and individual parameters (e.g., insulin sensitivity) may include:
1. Determining data representing the healthy response and the user's response to a certain meal type and graphically representing this data, as shown in FIG. 5.
2. Dividing the time of the response pattern into n segments (n being any positive number).
3. Computing the AUC (e.g., in min^{∗}mg/dL) of an average healthy response ("AUCₕ") in each segment.
4. Computing the AUC of the user's response ("AUCᵤ") in each segment.
5. Determining the ideal bolus dose distribution over time (i.e., delivery rate) from the difference between the AUC of the user and the non-diabetic "AUCₕ" (i.e., AUCᵤ-AUCₕ) for each segment, namely, the bolus dose per segment required to substantially match the user's BG to that of an average BG of a non-diabetic individual.

In FIG. 5 the curve is divided, for simplicity, into three segments (segments A, B, C) according to some embodiments. Segment A starts at t=40 min. due to the relative similarity in the curves of the user's response and the healthy response immediately after meal consumption. In some embodiments, a positive correction bolus dose (i.e., a dose in addition to what has been delivered as an extended bolus) or a negative correction bolus dose (i.e., less than what has been delivered) can be computed per segment. For segment A, (AUCᵤ₄₀₋₈₀-AUCₕ₄₀₋₈₀)/IS = Insulin ^{∗}t_{A}, [UI^{∗}min] where AUCₕ₄₀₋₈₀ may represent the AUC of the average healthy response between 40 and 80 minutes after the meal, AUCᵤ₄₀₋₈₀ is the area under the curve of the user's response between 40 and 80 minutes after the meal, t_{A} is the duration of time of segment A and IS is the insulin sensitivity of the user (i.e., the BG level lowered by one unit of insulin). The correction insulin dose for segment A therefore equals (AUCᵤ₄₀₋₈₀-AUCₕ₄₀₋₈₀)/(IS^{∗}t_{A}). For segment B, (AUCᵤ₈₀₋₁₂₀-AUCₕ₈₀₋₁₂₀)/IS = Insulin^{∗}t_{B}, where t_{B} is the duration of time of segment B. The correction insulin dose for segment B therefore equals (AUCᵤ₈₀₋₁₂₀-AUCₕ₈₀₋₁₂₀)/(IS^{∗}t_{B}). For segment C, (AUCᵤ₁₂₀₋₁₆₀-AUC₁₂₀₋₁₆₀)/IS = Insulin ^{∗}tc, where tc is the duration of time of segment C. The correction insulin dose for segment C therefore equals (AUCᵤ₁₂₀₋₁₆₀-AUC₁₂₀₋₁₆₀)/(IS^{∗}t_{C}).

According to some embodiments, the total bolus dose may be redistributed over the delivery time period according to the computed correction bolus doses for each segment. In some embodiments, the absolute bolus doses per segment may then be expressed as a percentage of the total bolus dose per segment, thus providing a bolus delivery pattern tailored to the user for the corresponding meal. Subsequent meals of similar type (e.g., similar dietary or nutritional content), even of different sizes, may be counteracted by bolus doses delivered according to the tailored bolus delivery pattern.

In some arrangements, bolus dose administration time may be adjusted and/or corrected in accordance with the pharmacodynamics of a specific insulin type . For example, if insulin Lispro is used, which has an onset time of approximately 15 minutes from administration, distribution of the bolus dose may be time shifted by 15 minutes to match the action of the insulin lispro. In some arrangements, the time shift may consider additional pharmacodynamics characteristics including without limitation the time until peak activity is obtained, the total duration of action of the delivered bolus dose.

Using the abovementioned procedure, a bolus delivery pattern may be tailored to the user based on meal type. The more segments into which a pattern is divided and the more BG measurements performed, the more accurate the tailored pattern will become. With that said, however, the utility of additional segments in accurately tailoring a bolus delivery pattern will depend on the state of the user and the specific meal type. For example, not all meals may precisely match a pre-established bolus delivery pattern.

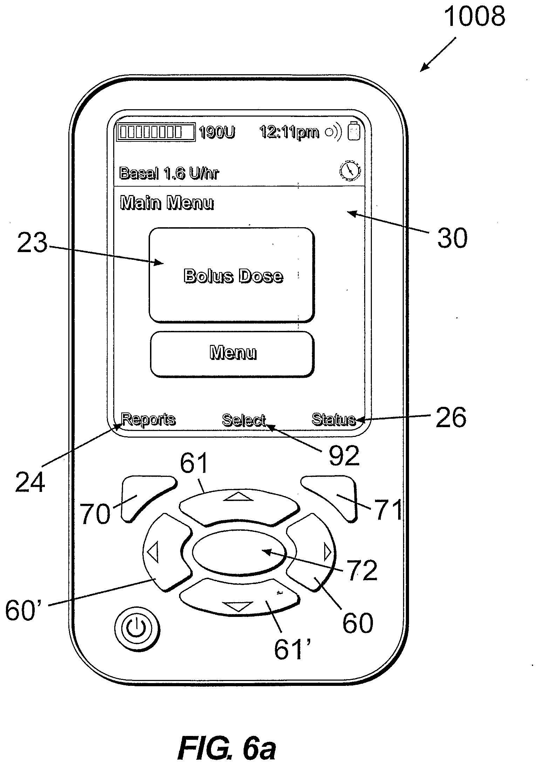

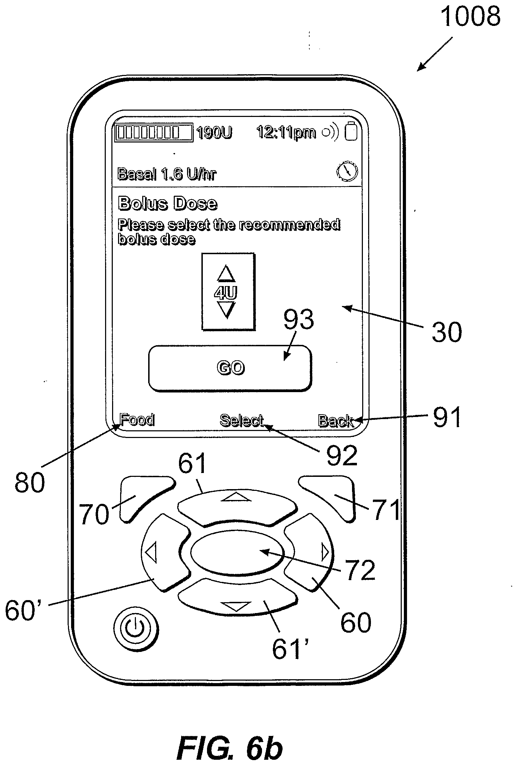

FIGS. 6a-d show user interfaces for selecting bolus doses and tailored bolus delivery patterns. In some arrangements, the user interface may be displayed on a screen 30 of the remote control unit 1008, a screen on the dispensing patch unit 1010 (not shown) or screens on both remote control unit 1008 and dispensing patch unit 1010. The user interface can be provided with a plurality of navigation windows for data input according to some arrangements. For example, FIG. 6a shows an example of a main window of the user interface. If the user selects "Bolus Dose" 23, then the window shown in FIG. 6b can be displayed. In some arrangements, auxiliary windows may be provided and/or selected by pressing, for example, soft keys 70 and/or 71. Some arrangements may have a window for downloading last bolus dose data displayed by selecting the "Reports" function 24 (shown in FIG. 6a). In response to selecting the "Status" function 26, the user may display data regarding ongoing bolus doses. The user may also display data regarding analyte (e.g., glucose) concentration levels by selecting the corresponding function.

FIG. 6b also shows an example of a window which can be used for selection of a bolus dose. According to some arrangements, the bolus dose can be recommended by the insulin infusion device 1000 itself based on personal parameters (e.g., CIR, IS, CBG or TBG) associated with a specific user. Types of input data used for determining the bolus dose are described, for example, in co-owned, co-pending U.S. publication no. 2008/0234663, the content of which is incorporated herein by reference in its entirety. According to some arrangements, the most frequently used and/or an averaged bolus dose level, selected during a specific time interval, can be presented at this window and presented as a first or "default" choice. This feature may be especially beneficial for users with routine daily intakes. In some arrangements, the "Food" function 80 may also be provided. Selecting this function using, for example, the soft key 70, can provide the user with a food database displaying different types of foods and their GI and/or carbohydrate load and/or fat content.

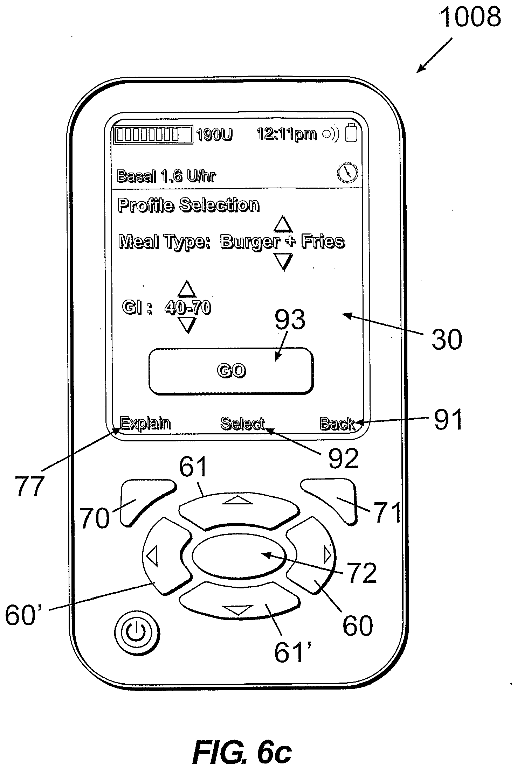

FIG. 6c shows an example of a window in which the user selects the relevant meal type for subsequent adjustments of the optimal bolus delivery pattern. In certain arrangements, the meal type can be described in terms of meal type or GI range. In some arrangements, the "Explain" function 77 can also be provided. Selecting this function using, for example, soft key 70, can provide the user with a short, general explanation of the meal content and its relevance to one of the pre-established tailored bolus delivery pattern.

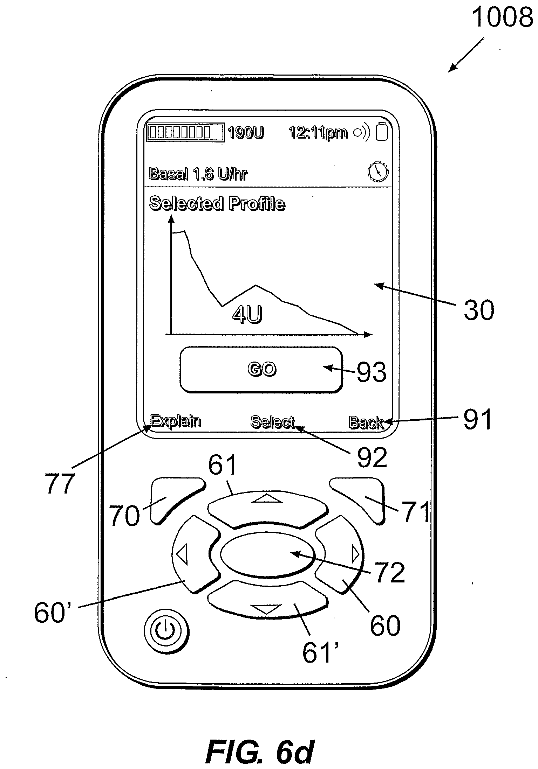

In a subsequent window, depicted in FIG. 6d, the pre-established tailored bolus delivery pattern associated with the meal type selected in the window depicted in FIG. 6c and the dose selected in FIG. 6b, may be graphically displayed according to some arrangements. The AUC of the displayed bolus delivery pattern represents the bolus dose that needs to be delivered. This user interface also illustrates the "GO," "Explain" and "Back" functions. In some arrangements, selecting the "GO" function may initiate delivery of a bolus dose (e.g., the insulin bolus) according to data represented graphically in the user interface display.

According to some arrangements, the user may be prompted to select a meal-type dependent pattern via the window displays depicted in FIGS. 6c and 6d and to initiate bolus dose delivery using the "GO" function shown in FIG. 6d. In some arrangements, applying the tailored bolus delivery pattern feature is not required to initiate a bolus dose delivery. That is, in some arrangements, a bolus dose does not have to be delivered according to a bolus delivery pattern, tailored or otherwise.

In some arrangements, where the user interface is provided in the remote control unit 1008, the user can navigate between displayed icons using horizontal 60, 60' and vertical 61, 61' buttons. Selection of a particular bolus dose and/or a bolus delivery pattern may be made by scrolling up and down through a list of possible doses and patterns to choose from, according to some arrangements. The user may also press the soft keys, namely, left key 70, right key 71 and center key 72 of the remote control unit 1008. These soft keys, when pressed, function to select certain functions on the display. In the given example of FIG. 6c, upon pressing the left soft key 70, an "Explain" function 77 is activated. Upon pressing the right soft key 71, the "Back" function 91, which transfers the display to the previous entered window, is performed. Upon pressing the central soft key 70, the "GO" function 93 is performed and transfers the user to the next window.

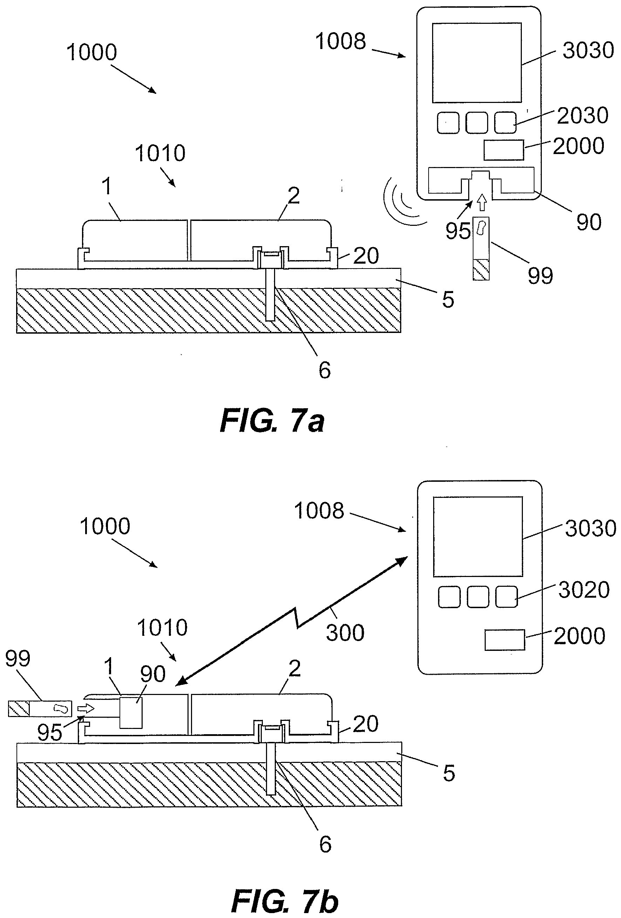

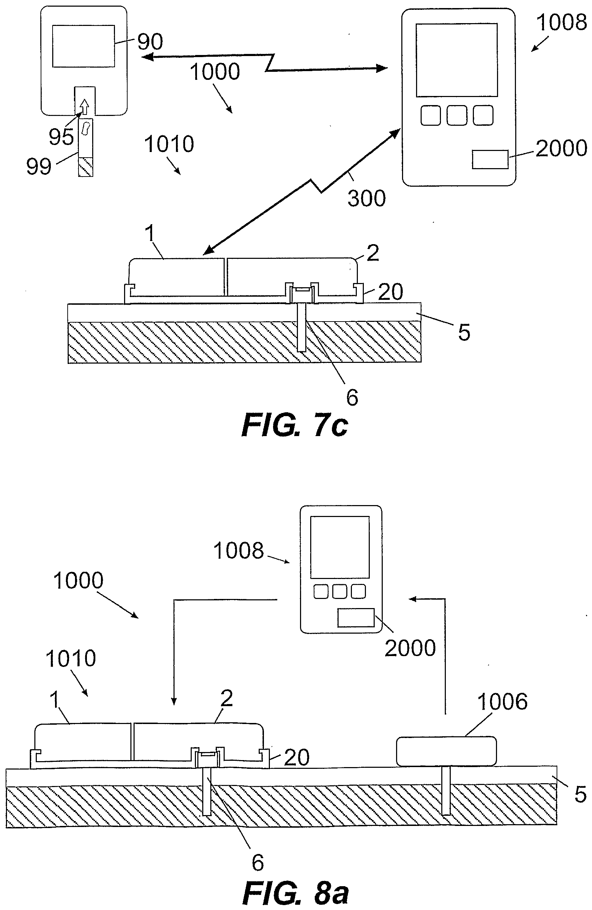

FIGS. 7a-c show schematic diagrams of three different arrangements of an insulin infusion device 1000. The insulin infusion device 1000 may include a dispensing patch unit 1010, a remote control unit 1008 implemented with a functionality to provide a tailored bolus delivery pattern and a sensor 90 (e.g., "glucometer") to perform BG monitoring for providing a tailored bolus delivery pattern. FIG. 7a shows a sensor 90 located in the remote control unit 1008. In some arrangements, the sensor 90 may comprise an opening 95 for receiving of a test strip 99. The user may extract blood from the body, place a blood drop on the test strip 99 and insert the strip 99 into the opening 95, according to some arrangements. The glucose readings may be shown on a display 3030 of the remote control unit 1008. FIG. 7b shows a sensor 90 located in the reusable part 1 of the dispensing patch unit 1010, according to some arrangements. FIG. 7c shows an arrangement in which glucose readings are directly or remotely received from an independent sensor 90.

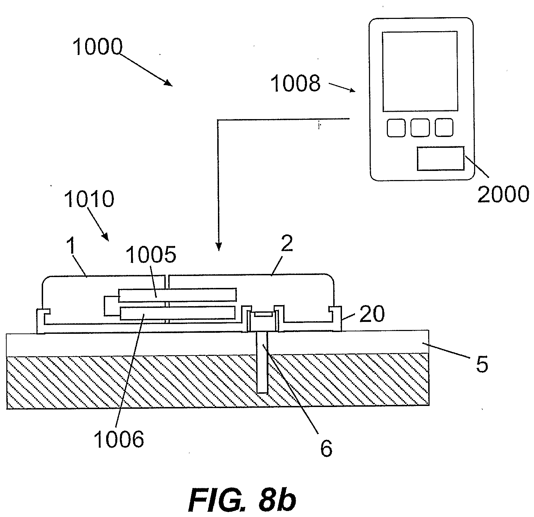

FIGS. 8a-b show schematic diagrams of two embodiments of an insulin infusion device 1000. The insulin infusion device 1000 may include a dispensing patch unit 1000, a remote control unit 1008 configured to include a tailored bolus delivery pattern module 2000 and a continuous subcutaneous glucose monitor 1006 required for the glucose measurements needed for performing the tailored bolus delivery pattern procedure. Specifically, FIG. 8a shows an embodiment in which the current BG concentration can be received from an independent continuous subcutaneous glucose monitor 1006. FIG. 8b shows an embodiment in which the continuous subcutaneous glucose monitor 1006 is located in the dispensing patch unit 1010 of the insulin infusion device 1000. As disclosed in co-owned, co-pending PCT Application Nos, PCT/IL07/000163, PCT/IL07/001579, and PCT/IL08/001521, published res[ectively as WO 2007/093981, WO 2008/078319 and WO 2009/066288, the insulin dispensing apparatus 1005 and continuous subcutaneous glucose monitor 1006 shown in FIG. 8b may constitute, in the illustrated embodiment, a common insulin delivery device and share a single cannula 6 for both dispensing and sensing according to some embodiments. In some embodiments, the continuous subcutaneous glucose monitor 1006 and the insulin dispensing apparatus 1005 may have separate cannulae that penetrate the skin 5 and reside in the subcutaneous tissue.

In some arrangements the device 1000 can be configured to operate as a semi- closed loop system. In a semi-closed system, the feedback and control between sensor measurements and associated therapeutic fluid release can be partially automatic. For example, the release of therapeutic fluid in a basal rate can be automatically controlled by a processor based on analytes measurements, while the release of bolus doses of therapeutic fluid may be delivered according to a user's input. In some arrangements, insulin can automatically be dispensed according to continuous monitoring of glucose levels and according to additional pre- meal bolus user inputs (semi-closed loop). The tailored bolus delivery pattern module 2000 shown in FIG. 8b can be used for bolus inputs in the semi-closed loop system.

Various embodiments of the subject matter described herein may be realized in digital electronic circuitry, integrated circuitry, specially designed ASICs (application specific integrated circuits), computer hardware, firmware, software, and/or combinations thereof. These various embodiments may include being implemented in one or more computer programs that are executable and/or interpretable on a programmable system including without limitation (i) at least one programmable processor, which may be special or general purpose, coupled to receive data and instructions from, and to transmit data and instructions to, a storage system, (ii) at least one input device and/or (iii) at least one output device.

These computer programs (also known as programs, software, software applications or code) may include machine instructions for a programmable processor and may be implemented in a high-level procedural and/or object-oriented programming language and/or in assembly/machine language. As used herein, the term "machine-readable medium" refers to any computer program product, apparatus and/or device (e.g., magnetic discs, optical disks, memory or Programmable Logic Devices (PLDs)) used to provide machine instructions and/or data to a programmable processor, including without limitation a machine-readable medium that receives machine instructions as a machine-readable signal. The term "machine-readable signal" refers to any signal used to provide machine instructions and/or data to a programmable processor.

To provide for interaction with a user, the subject matter described herein may be implemented on a computer having a display device (e.g., a CRT (cathode ray tube) or LCD (liquid crystal display) monitor) for displaying information to the user, a keyboard and a pointing device (e.g., a mouse or a trackball) for providing input to the computer. Other types of devices may also be used to enable interaction with a user. For example, output to the user may be provided in any form, including without limitation sensory feedback (e.g., visual feedback, auditory feedback or tactile feedback) and input from the user may be received in any form, including without limitation acoustic, speech or tactile input.

The subject matter described herein may be implemented in a computing system that includes a back-end component (e.g., as a data server), a middleware component (e.g., an application server) a front-end component (e.g., a client computer having a graphic interface or a Web browser through which a user may interact with the subject matter described herein), or any combination of back-end, middleware or front-end components. The components of the system may be interconnected by any form of digital data communication (e.g., a communication network). Examples of communication networks include a local area network ("LAN"), a wide area network ("WAN") and the Internet.

The computing system may also include clients and servers. A client and server are generally remote from each other and typically interact through a communication network. The relationship of client and server may arise by virtue of computer programs running on the respective computers and having a client-server relationship.

Example embodiments of the methods and devices of the present disclosure have been described herein. As noted elsewhere, these embodiments have been described for illustrative purposes only and are not limiting. Other embodiments are possible and are covered by the present disclosure. For example, the logic flow depicted in the accompanying figures and described herein do not require the particular order shown, or sequential order, to achieve desirable results. Such embodiments will be apparent to persons of ordinary skill in the relevant art(s) based on the teachings contained herein. Thus, the breadth and scope of the disclosure should not be limited by any of the above-described embodiments but should be defined only in accordance with the following claims.

## Claims

1. A therapeutic fluid dispensing device (1000) to deliver therapeutic fluid into the body of a patient, the device comprising: a controller (2010) to determine a bolus delivery pattern based on a specific dietary intake of the patient; and an infusion pump to deliver the therapeutic fluid into the body of the patient based, at least in part, on the determined bolus delivery pattern, wherein the controller (2010) is configured to select the bolus delivery pattern from a plurality of bolus delivery patterns, **characterized in that**:
the device further comprises a blood glucose sensor (90) to measure the blood glucose level of the patient, with the blood glucose sensor including a continuous glucose monitor (1006);
at least one of the bolus delivery patterns is determined based on corresponding blood glucose responses of one or more non-diabetic individuals that have consumed dietary intakes similar to the specific dietary intake of the patient; and
the controller (2010) is further configured:
to determine the bolus delivery patterns based additionally on blood glucose responses of the patient after the patient has consumed dietary intakes similar to the specific dietary intakes, the blood glucose responses of the patient being measured by the continuous glucose monitor (1006);
to compute a difference between an area under the curve of a graphical representation of the response of the patient to a respective type of dietary intake and an area under the curve of a graphical representation of the response of the one or more non- diabetic individuals to the respective type of dietary intake; and
to determine a tailored bolus dose distribution over time from the difference computed.

2. The device (1000) of claim 1, further comprising an electronic storage device (2000) to store the one or more bolus delivery patterns.

3. The device (1000) of claim 1, wherein the specific dietary intake comprises a glycemic index representative of the effect of the carbohydrate content of food corresponding to the type of dietary intake on blood glucose levels in individuals consuming the food.

4. The device (1000) of claim 1, further comprising a user-input interface (2030) to receive information regarding the type of dietary intake.

5. The device (1000) of claim 1, further comprising a reservoir in fluid communication with the infusion pump, the reservoir configured to contain the therapeutic fluid.

6. The device (1000) of claim 1, further comprising a display (2040) to represent graphically the determined bolus dose delivery pattern.

7. The device (1000) of claim 1, wherein the infusion pump is configured to deliver the therapeutic fluid into the body of the patient based on at least one of the determined bolus delivery pattern and the measured blood glucose level.

8. The device (1000) of claim 1, wherein the continuous glucose monitor is disposed in a dispensing patch unit (1010) that includes the infusion pump.

9. A method of tailoring a bolus delivery pattern for a user, comprising:
measuring with a continuous glucose monitor (1006) a user's response to a known meal consumed with a known bolus dose delivered according to a selected pre-established bolus delivery pattern appropriate for the known meal;
plotting (403) as a blood glucose versus time curve the user's response to the known meal consumed with the known bolus dose delivered according to the selected pre-established bolus delivery pattern appropriate for the known meal;
comparing (404) the user's response to the known meal to the response of a healthy (non-diabetic) individual for a meal of the same type as the known meal in order to compute a difference between an area under the curve of a graphical representation of the user's response to the known meal and an area under the curve of a graphical representation of the response of the healthy (non- diabetic) individual to the meal of the same type as the known meal; and
tailoring (405) the pre-established bolus delivery pattern to the user using the difference computed so that the user's response will substantially match, or nearly match, the response of the healthy (non-diabetic) individual.

## Patentansprüche

1. Ausgabevorrichtung (1000) eines therapeutischen Fluids zum Verabreichen eines therapeutischen Fluids in den Körper eines Patienten, wobei die Vorrichtung Folgendes umfasst: eine Steuerung (2010), um ein Bolusverabreichungsmuster auf der Basis einer spezifischen Nahrungsaufnahme des Patienten zu bestimmen; und eine Infusionspumpe zum Verabreichen des therapeutischen Fluids in den Körper des Patienten mindestens teilweise auf der Basis des bestimmten Bolusverabreichungsmusters, wobei die Steuerung (2010) dazu konfiguriert ist, das Bolusverabreichungsmuster aus einer Vielzahl von Bolusverabreichungsmustern auszuwählen, **dadurch gekennzeichnet, dass**:
die Vorrichtung ferner einen Blutzuckersensor (90) zum Messen des Blutzuckerspiegels des Patienten umfasst, wobei der Blutzuckersensor eine Vorrichtung (1006) zur ununterbrochenen Glukoseüberwachung einschließt;
mindestens eines der Bolusverabreichungsmuster auf der Basis von entsprechenden Blutzuckerreaktionen von einem oder mehreren nichtdiabetischen Individuen bestimmt wird, die Nahrungsaufnahmen konsumiert haben, die der spezifischen Nahrungsaufnahme des Patienten ähneln; und wobei die Steuerung (2010) ferner dazu konfiguriert ist:
die Bolusverabreichungsmuster zusätzlich auf der Basis von Blutzuckerreaktionen des Patienten zu bestimmen, nachdem der Patient Nahrungsaufnahmen konsumiert hat, die den spezifischen Nahrungsaufnahmen ähneln, wobei die Blutzuckerreaktionen des Patienten durch eine Vorrichtung (1006) zur ununterbrochenen Glukoseüberwachung gemessen werden;
einen Unterschied zwischen einem Bereich unterhalb der Kurve einer grafischen Darstellung der Reaktion des Patienten auf eine jeweilige Art von Nahrungsaufnahme und einem Bereich unterhalb der Kurve einer grafischen Darstellung der Reaktion des einen oder der mehreren nichtdiabetischen Individuen auf die jeweilige Art von Nahrungsaufnahme zu berechnen;
und eine zugeschnittene Bolusdosisverteilung über eine Zeitspanne aus dem berechneten Unterschied zu bestimmen.

2. Vorrichtung (1000) nach Anspruch 1, ferner umfassend eine elektronische Speichervorrichtung (2000) zum Speichern des einen oder der mehreren Bolusverabreichungsmuster.

3. Vorrichtung (1000) nach Anspruch 1, wobei die spezifische Nahrungsaufnahme einen glykämischen Index umfasst, der die Wirkung des Kohlehydratgehalts von Lebensmitteln, die der Art der Nahrungsaufnahme entsprechen, auf Blutzuckerspiegel von Individuen, die die Lebensmittel konsumieren, darstellt.

4. Vorrichtung (1000) nach Anspruch 1, ferner umfassend eine Benutzereingabe-Schnittstelle (2030) zum Empfangen von Informationen, die sich auf die Art der Nahrungsaufnahme beziehen.

5. Vorrichtung (1000) nach Anspruch 1, ferner umfassend einen Behälter, der in Fluidkommunikation mit der Infusionspumpe steht, wobei der Behälter dazu konfiguriert ist, das therapeutische Fluid zu enthalten.

6. Vorrichtung (1000) nach Anspruch 1, ferner umfassend eine Anzeige (2040) zum grafischen Darstellen des bestimmten Bolusdosisverabreichungsmusters.

7. Vorrichtung (1000) nach Anspruch 1, wobei die Infusionspumpe dazu konfiguriert ist, das therapeutische Fluid auf der Basis von mindestens einem von dem bestimmten Bolusverabreichungsmuster und dem gemessenen Blutzuckerspiegel zu verabreichen.

8. Vorrichtung (1000) nach Anspruch 1, wobei die Vorrichtung zur ununterbrochenen Glukoseüberwachung in einer Ausgabe-Patch-Einheit (1010) angeordnet ist, die die Infusionspumpe einschließt.

9. Verfahren zum Zuschneiden eines Bolusverabreichungsmusters für einen Benutzer, wobei das Verfahren Folgendes umfasst:
Messen mit einer Vorrichtung (1006) zur ununterbrochenen Glukoseüberwachung einer Benutzerreaktion auf eine bekannte Mahlzeit, die mit einer bekannten Bolusdosis konsumiert wird, die gemäß einem ausgewählten voretablierten Bolusverabreichungsmuster verabreicht wird, das für die bekannte Mahlzeit angemessen ist;
Aufzeichnen (403) der Benutzerreaktion auf die bekannte Mahlzeit, die mit der bekannten Bolusdosis konsumiert wird, die gemäß dem ausgewählten voretablierten Bolusverabreichungsmuster verabreicht wird, das für die bekannte Mahlzeit angemessen ist, als eine Blutzucker-versus-Zeit-Kurve;
Vergleichen (404) der Benutzerreaktion auf die bekannte Mahlzeit mit der Reaktion eines gesunden (nichtdiabetischen) Individuums für eine Mahlzeit derselben Art wie die bekannte Mahlzeit, um einen Unterschied zwischen einem Bereich unterhalb der Kurve einer grafischen Darstellung der Benutzerreaktion auf die bekannte Mahlzeit und einem Bereich unterhalb der Kurve einer grafischen Darstellung der Reaktion des gesunden (nichtdiabetischen) Individuums auf die Mahlzeit derselben Art wie die bekannte Mahlzeit zu berechnen; und
Zuschneiden (405) des voretablierten Bolusverabreichungsmusters auf den Benutzer unter Verwendung des berechneten Unterschieds, sodass die Benutzerreaktion im Wesentlichen mit der Reaktion des gesunden (nichtdiabetischen) Individuums übereinstimmt oder annährend übereinstimmt.

## Revendications

1. Dispositif de distribution d'un fluide thérapeutique (1000) pour délivrer un fluide thérapeutique dans le corps d'un patient, le dispositif comprenant : une unité de commande (2010) pour déterminer un modèle de délivrance de bolus sur la base d'un apport nutritionnel spécifique du patient ; et une pompe à perfusion pour délivrer le fluide thérapeutique dans le corps du patient sur la base, au moins en partie, du modèle de délivrance de bolus déterminé, dans lequel l'unité de commande (2010) est configurée pour sélectionner le modèle de délivrance de bolus parmi une pluralité de modèles de délivrance de bolus, **caractérisé en ce que** :
le dispositif comprend en outre un capteur de glycémie (90) pour mesurer le niveau de glycémie du patient, avec le capteur de glycémie incluant un système de mesure de glycémie en continu (1006) ;
au moins l'un des modèles de délivrance de bolus est déterminé sur la base de réponses de glycémie correspondantes d'une ou plusieurs personnes non-diabétiques qui ont consommé des apports nutritionnels similaires à l'apport nutritionnel spécifique du patient ; et l'unité de commande (2010) est en outre configurée pour :
déterminer les modèles de délivrance de bolus sur la base également de réponses de glycémie du patient après que le patient a consommé des apports nutritionnels similaires aux apports nutritionnels spécifiques, les réponses de glycémie du patient étant mesurées par le système de mesure de glycémie en continu (1006) ;
calculer une différence entre une zone sous la courbe d'une représentation graphique de la réponse du patient à un type respectif d'apport nutritionnel et une zone sous la courbe d'une représentation graphique de la réponse de l'une ou plusieurs personnes non-diabétiques au type respectif d'apport nutritionnel ;
et déterminer une distribution de dose de bolus personnalisé dans le temps à partir de la différence calculée.

2. Dispositif (1000) selon la revendication 1, comprenant en outre un dispositif de stockage électronique (2000) pour stocker l'un ou plusieurs modèles de délivrance de bolus.

3. Dispositif (1000) selon la revendication 1, dans lequel l'apport nutritionnel spécifique comprend un indice glycémique représentatif de l'effet de la teneur en hydrates de carbone des aliments correspondant au type d'apport nutritionnel sur les niveaux de glycémie chez les personnes consommant les aliments.

4. Dispositif (1000) selon la revendication 1, comprenant en outre une interface d'entrée utilisateur (2030) pour recevoir des informations concernant le type d'apport nutritionnel.

5. Dispositif (1000) selon la revendication 1, comprenant en outre un réservoir en communication fluide avec la pompe à perfusion, le réservoir configuré pour contenir le fluide thérapeutique.

6. Dispositif (1000) selon la revendication 1, comprenant en outre un affichage (2040) pour représenter graphiquement le modèle de délivrance de dose de bolus déterminé.

7. Dispositif (1000) selon la revendication 1, dans lequel la pompe à perfusion est configurée pour délivrer le fluide thérapeutique dans le corps du patient sur la base d'au moins l'un du modèle de délivrance de bolus déterminé et du niveau de glycémie mesuré.

8. Dispositif (1000) selon la revendication 1, dans lequel le système de mesure de glycémie en continu est disposé dans une unité de timbre de distribution (1010) qui inclut la pompe à perfusion.

9. Procédé de personnalisation d'un modèle de délivrance de bolus pour un utilisateur, comprenant :
la mesure avec un système de mesure de glycémie en continu (1006) d'une réponse d'utilisateur à un repas connu consommé avec une dose de bolus connue délivrée en fonction d'un modèle de délivrance de bolus pré-établi sélectionné approprié pour le repas connu ;
le tracé (403) sous la forme d'une courbe de glycémie par rapport au temps de la réponse d'utilisateur au repas connu consommé avec la dose de bolus connue délivrée en fonction du modèle de délivrance de bolus pré-établi sélectionné approprié pour le repas connu ;
la comparaison (404) de la réponse d'utilisateur au repas connu à la réponse d'une personne en bonne santé (non-diabétique) pour un repas du même type que le repas connu afin de calculer une différence entre une zone sous la courbe d'une représentation graphique de la réponse d'utilisateur au repas connu et une zone sous la courbe d'une représentation graphique de la réponse de la personne en bonne santé (non-diabétique) au repas du même type que le repas connu ;
et la personnalisation (405) du modèle de délivrance de bolus pré-établi à l'utilisateur en utilisant la différence calculée de façon à ce que la réponse d'utilisateur corresponde sensiblement, ou corresponde pratiquement, à la réponse d'une personne en bonne santé (non-diabétique).
